# EUROPÄISCHE PATENTSCHRIFT

(11) **EP 3 897 758 B1**
(45) Veröffentlichungstag und Bekanntmachung des Hinweises auf die Patenterteilung: **01.03.2023**
(21) Anmeldenummer: 19828737.7
(22) Anmeldetag: 19.12.2019
(51) Int. Cl.: A61L 15/18, A61L 15/44

(54) **MITTEL ZUR BEHANDLUNG BLUTENDER WUNDEN**
AGENT FOR TREATING BLEEDING WOUNDS
MILIEU DE TRAITEMENT DES PLAIES HÉMORRAGIQUES

(30) Priorität: 20.12.2018 EP 18214785; 23.08.2019 EP 19193416
(43) Veröffentlichungstag der Anmeldung: 27.10.2021
(73) Patentinhaber: BK Giulini GmbH, 68526 Ladenburg (DE); FKuR Property GmbH, 47877 Willich (DE)
(72) Erfinder: STAFFEL, Thomas, 67269 Grünstadt (DE); THAUERN, Henrike, 69469 Weinheim (DE); STRAUB, Juergen, 68305 Mannheim (DE); DOLFEN, Edmund, 47877 Willich (DE); MICHELS, Carmen, 47877 Willich (DE); KRPAN, Karel, 47877 Willich (DE); NEUMANN, Frank-Martin, 47877 Willich (DE)
(74) Vertreter: Reitstötter Kinzebach
(86) Internationale Anmeldenummer: PCT/EP2019/086325
(87) Internationale Veröffentlichungsnummer: WO 2020/127745

(56) Entgegenhaltungen:
- WO-A1-2015/063190

## Beschreibung

Die vorliegende Erfindung betrifft Mittel zur Behandlung blutender Wunden von Säugern, insbesondere zur Behandlung blutender Wunden von Menschen und speziell von Patienten, deren Blutgerinnung durch Verabreichung gerinnungshemmender Mittel beeinflusst wurde.

Durch Verletzungen von menschlichen und tierischen Gewebeschichten können darin befindliche Blutgefäße beschädigt werden, was zu Blutaustritt an der betroffenen Stelle auf der Haut oder im Körperinneren führen kann. Nach einer Phase des Blutaustritts setzt die natürliche Blutgerinnung ein. Die Blutgerinnung bezeichnet das Erstarren des flüssigen Blutes als physiologischen Schutzmechanismus. Die Blutgerinnung basiert auf zwei komplexen, kaskadenartig ablaufenden enzymatischen Reaktionswegen, an denen mehrere Gerinnungsfaktoren mitwirken. Beide Wege, die üblicherweise als intrinsischer und extrinsischer Weg bezeichnet werden, münden in die Bildung unlöslicher Fibrinfäden, die maßgeblich am Zustandekommen von wundschließenden Blutgerinnseln beteiligt sind. Thrombin, auch Gerinnungsfaktor IIa genannt, nimmt hierbei eine Schlüsselstellung ein, da es nicht nur die Bildung von Fibrin bewirkt, sondern auch durch positive Rückkopplung an mehreren Stellen der Gerinnungskaskade aktivierend eingreift. Faktor XII, auch als Hagemann-Faktor bezeichnet, wiederum steht am Anfang des intrinsischen Wegs der Gerinnungskaskade und triggert letztendlich die Bildung von Thrombin aus Prothrombin.

Die natürliche Blutgerinnung setzt zwar fast unmittelbar nach Eintritt des Traumas ein, ist aber ein relativ langsamer Prozess, der zum Verschließen der Wunde und damit einhergehender Blutstillung einige Zeit beansprucht. In vielen Fällen ist daher eine beschleunigte Blutstillung wünschenswert, beispielsweise aus hygienischen Gründen oder wenn aufgrund der Größe der Wunde oder der Stärke des Blutflusses ein übermäßiger Blutverlust zu befürchten ist. Altbekannte Methoden zur Blutstillung, wie etwa Abdecken der Wunde, Anlegen von Druckverbänden und Klammern oder Nähen der Wunde, sind oftmals unzureichend und insbesondere bei Verletzung der Gefäße innerer Organe nicht oder nur schwierig möglich. Aus dem Stand der Technik sind daher Verfahren bekannt, die zur Verbesserung der Blutstillung beitragen sollen, beispielsweise das Einbringen blutgerinnungsaktiver Substanzen wie Fibrinkleber in den Wundbereich.

Verschiedentlich wurde der Einsatz anorganischer Substanzen als blutstillende Mittel beschrieben. So beschreiben beispielsweise US 4,822,349 und US 2003/0133990 den Einsatz von Mitteln auf der Basis dehydratisierter Zeolithe zur Behandlung blutender Wunden. Es wird postuliert, dass der dehydratisierte Zeolith die Blutgerinnung durch Absorption von Wasser aus dem austretenden Blut auslöst und die dabei freiwerdende Wärme den Wundverschluss fördert. Derartige Mittel wurden zeitweise unter der Handelsbezeichnung QuickClot^{®} vertrieben. Bei der Anwendung kam es jedoch verschiedentlich zu schweren Gewebeschädigungen (siehe J. K. Wright et al., J. Trauma 57 (2004), 224-230). Eine Verringerung der Nachteile von Mitteln auf Basis dehydratisierter Zeolithe wird durch die in US 2006/0039994 beschriebenen Molekularsiebe auf Basis von Aluminiumphosphat erzielt.

WO 2006/088912 beschreibt die blutstillende Wirkung von Tonmineralien wie Bentonit oder Kaolin. Entsprechende blutstillende Mittel auf der Basis von Kaolin wurden unter der Handelsbezeichnung Woundstat^{®} vermarktet. Man nimmt auch hier an, dass die Blutgerinnung durch Absorption von Wasser aus dem austretenden Blut ausgelöst wird und die in diesen Mitteln ebenfalls enthaltene Polyacrylsäure einen mechanischen Wundverschluss bewirkt. Diese Mittel werden jedoch aufgrund von aufgetretenen Gewebeschäden und dem Auftreten von Embolien nicht mehr eingesetzt (siehe G. Lawton et al., JR. Army Med. Corps 155(4) (2009) 309-314 mit weiteren Nachweisen).

WO 2015/063190 beschreibt die blutstillende Wirkung von kristallinen, wasserunlöslichen anorganischen Polyphosphaten. Man nimmt an, dass die hämostatische Wirkung dieser Polyphosphate auf einer Kontaktaktivierung der Gerinnungskaskade beruht, die vermutlich über den Faktor XII vermittelt wird, welcher seinerseits die Thrombinbildung und damit die Bildung von Fibrin aus Fibrinogen über den intrisischen Weg auslöst. Die Applikation dieser Polyphosphate erfolgt typischerweise in Form eines Pulvers .

Bei blutenden Wunden ist die Handhabung von partikulären Substanzen wie Pulvern jedoch problematisch. So besteht die Gefahr, dass die Substanz bei starken Blutungen schnell aus dem Wundgebiet weggeschwemmt und somit unwirksam wird, während sie bei schwächeren Blutungen in die Blutbahn gelangen kann und unerwünschte Auswirkungen an von der Wunde weit entfernten Stellen des Körpers haben kann, wie beispielsweise die Bildung von thrombotischen Blutgerinnseln.

Die Aufgabe der Erfindung liegt daher in der Bereitstellung eines gut handhabbaren Mittels mit blutstillender Wirkung, das die Nachteile des Standes der Technik nicht aufweist. Das Mittel sollte sowohl bei leicht wie auch stark blutenden Wunden am Menschen eingesetzt werden können, insbesondere bei operativ bedingten Wunden und/oder bei Patienten, deren Blutgerinnung durch Verabreichung gerinnungshemmender Mittel herabgesetzt wurde. Das Mittel soll effizient die dabei auftretenden Blutungen stillen. Zudem sollte es sich einfach und kostengünstig herstellen lassen und in der medizinischen Praxis mittels üblicher Methoden leicht und zuverlässig anwendbar sein. Überraschenderweise geht die blutstillende Wirkung von kristallinen, wasserunlöslichen anorganischen Polyphosphaten, wie sie in der WO 2015/063190 beschrieben werden, nicht verloren, wenn sie in fein verteilter Form in eine Polymermatrix eingebettet sind. Dies erlaubt beispielsweise die Herstellung von Pads oder Folien mit blutstillender Wirkung, die sich leichter als entsprechende Pulver auf blutende Wunden von Säugern, insbesondere von Menschen, applizieren lassen.

Dies überrascht, da man, wie bereits erwähnt, davon ausgehen muss, dass die blutstillende Wirkung der wasserunlöslichen anorganischen Polyphosphate auf einer Kontaktaktivierung des Faktors XII basiert.

Dementsprechend betrifft die vorliegende Erfindung Mittel zur Verwendung zur Behandlung blutender Wunden von Säugern in Form flexibler Flächengebilde, insbesondere in Form von Pads oder Folien, die aus einem organischen Polymermaterial gebildet werden, welches wenigstens ein partikuläres, kristallines anorganisches Polyphosphat enthält, wobei das organische Polymermaterial eine flächenförmige Matrix bildet, in welcher die Partikel des partikulären, kristallinen, anorganischen Polyphosphats in fein verteilter Form vorliegen, wobei das Polyphosphat bei 20 °C eine Löslichkeit in entionisiertem Wasser von weniger als 5 g/L, insbesondere weniger als 1 g/L aufweist, wobei das Anion des Polyphosphats im Mittel (Zahlenmittel) mindestens vier Phosphoratome pro Polyphosphat-Anion aufweist.

Die Erfindung betrifft insbesondere derartige Mittel zur Verwendung zur Behandlung blutender Wunden von Menschen und speziell von Patienten, deren Blutgerinnung durch Verabreichung gerinnungshemmender Mittel beinflusst wurde.

Unter Flächengebilden versteht man grundsätzlich makroskopische, flächenförmige Gebilde mit Flächen von in der Regel wenigstens 1 cm², insbesondere wenigstens 2 cm² oder wenigstens 3 cm². Die Flächenobergrenze ist grundsätzlich von untergeordneter Bedeutung. Für die erfindungsgemäße Verwendung wird die Fläche des Gebildes in der Regel einen Wert von 2000 cm², und speziell 1000 cm² nicht überschreiten. Sie liegt insbesondere im Bereich von 1 bis 2000 cm² oder im Bereich von 2 bis 1500 cm² oder im Bereich von 3 bis 1000 cm². Die Dicke kann über breite Bereiche variert werden und liegt, je nach Art des Flächengebildes, im Bereich von 1 µm bis 20 mm, insbesondere im Bereich von 10 µm bis 10 mm. Die Bestimmung der Dicke kann in an sich bekannter Weise bestimmt werden. Die hier angegebenen Werte sind Mittelwerte der Dickenwerte, die an wenigstens fünf verschiedenen Prüfpunkten des Flächenmaterials bestimmt wurden.

Erfindungsgemäße Flächengebilde sind flexibel, d. h. sie lassen sich unter Einwirkung von mechanischen Kräften zumindest bezüglich ihrer Flächennormale elastisch verformen. Diese Verformbarkeit wird dadurch gewährleistet, dass das Flächengebilde von einem Polymermaterial gebildet wird, welches in Bezug auf die Abmessung, insbesondere dessen Dicke, eine geeignete Elastizität aufweist. Das Elastizitätsmodul typischer Polymermaterialien liegt typischerweise bei 20 °C im Bereich von 0,001 bis 3,0 GPa, insbesondere im Bereich von 0,1 bis 1,2 GPa.

Erfindungsgemäß werden die Flächengebilde von einem organischen Polymermaterial gebildet, in welchem das partikuläre, kristalline anorganische Polyphosphat in fein verteilter Form vorliegt. Anders als in textilen Flächengebilden, die aus Fasern aufgebaut sind, bildet in den erfindungsgemäßen Flächengebilden das organische, Polymermaterial eine flächenförmige Matrix, in welcher die Partikel des partikulären, kristallinen anorganischen Polyphosphats, im Folgenden Polyphosphat-Partikel, in feinverteilter Form vorliegen, bzw. in welcher die Polyphosphat-Partikel in fein verteilter Form eingebettet sind. Mit anderen Worten, das organischen Polymermaterial bildet eine kohärente Schicht, in welche die Polyphosphat-Partikel eingebettet sind. Hierbei können die Polyphosphat-Partikel vollständig oder teilweise von dem organischen Polymermaterial umhüllt werden.

Typische flexible Flächengebilde sind Folien und Pads. Unter Folien versteht man Flächengebilde mit einer Dicke von in der Regel nicht mehr als 1 mm, häufig nicht mehr als 500 µm, insbesondere nicht mehr als 250 µm oder 200 µm, z. B. Dicken im Bereich von 1 bis 1000 µm, häufig 5 bis 500 µm, insbesondere im Bereich von 10 bis 250 µm oder im Bereich von 20 bis 200 µm. Unter Pads versteht man kissenförmige Flächengebilde mit Dicken oberhalb 1 mm, z. B. Dicken im Bereich von 1 bis 20 mm, insbesondere im Bereich von 2 bis 10 mm.

Als Polyphosphate kommen grundsätzlich alle die in WO 2015/063190 beschriebenen kristallinen, anorganischen Polyphosphate in Betracht, die kristallin und im Wesentlichen wasserunlöslich sind, d. h. die bei 20 °C eine Löslichkeit in entionisiertem Wasser von weniger als 5 g/L, insbesondere weniger als 1 g/L aufweisen.

Die in den erfindungsgemäßen Flächengebilden enthaltenen anorganischen Polyphosphate sind kristallin und weisen in der Regel einen Kristallinitätsgrad von mindestens 90 %, vorzugsweise mindestens 95 % und insbesondere mindestens 98 % auf. Der Kristallinitätsgrad des Polyphosphats kann in an sich bekannter Weise mittels Röntgenpulverdiffraktometrie bestimmt werden, z. B. nach der in WO 2015/063190 beschriebenen Methode. Hierbei werden die Halbwertsbreiten der detektierten Reflexe der Diffraktogramme in an sich bekannter Weise zur Quantifizierung der Kristallinität herangezogen.

Die Polyphosphat-Anionen der in den erfindungsgemäßen Flächengebilden enthaltenen kristallinen, anorganischen Polyphosphate weisen typischerweise im Mittel (Zahlenmittel) wenigstens 6, insbesondere wenigstens 8 oder wenigstens 10 und speziell wenigstens 12 oder wenigstens 15 Phosphoratome pro Polyphosphat-Anion auf. Der Fachmann weiß, dass in derartigen Polyphosphaten die Polyphosphat-Anionen typischerweise nicht alle die gleiche Anzahl an Phosphoratomen pro Anion aufweisen, sondern sich in der Anzahl der Phosphoratome pro Polyphosphat-Anion unterscheiden. Insbesondere weisen die Polyphosphat-Anionen der erfindungsgemäßen Polyphosphate im Mittel (Zahlenmittel) 4 bis 2000, häufig 6 bis 1000 oder 8 bis 500, vorzugsweise 10 bis 400 oder 12 bis 300 und insbesondere 15 bis 200 Phosphoratome pro Polyphosphat-Anion auf. Die zahlenmittlere Anzahl der Phosphoratome im Polyphosphat-Anion kann in an sich bekannter Weise, z. B. durch ³¹P-Festkörper-NMR, bestimmt werden, beispielsweise mittels der in WO 2015/063190 beschriebenen Vorgehensweise.

Bevorzugt sind die kristallinen, anorganischen Polyphosphate unter kristallinen Alkalimetall-, Erdalkalimetall- und Ammoniumpolyphosphaten, die im Wesentlichen wasserunlöslich sind, ausgewählt. Bei den Kationen der kristallinen Polyphosphate handelt es sich dementsprechend vorzugsweise um Alkalimetall-, Erdalkalimetall- oder Ammoniumionen, die insbesondere unter Na⁺, K⁺, NH₄⁺, Ca²⁺ und Mg²⁺, und vorzugsweise unter Na⁺, K⁺, NH₄⁺ und Ca²⁺, ausgewählt sind.

Die Anionen der wasserunlöslichen kristallinen, anorganischen Polyphosphate sind in der Regel im Wesentlichen linear oder cyclisch, d. h. zu wenigstens 80 Mol-%, bevorzugt wenigstens 90 Mol-% und insbesondere wenigstens 95 Mol-%, bezogen auf die Gesamtmenge der Polyphosphatanionen im kristallinen Polyphosphat. Dementsprechend handelt es sich bei den Anionen derartiger Polyphosphate um Ketten aus meta-Phosphateinheiten PO₃⁻, in denen jeweils zwei Phosphoratome über ein Sauerstoffatom miteinander verbunden sind. Entsprechend wird hier unter "Kettenlänge" die Anzahl der im Polyphosphat-Anion enthaltenen meta-Phosphateinheiten PO₃⁻ verstanden. Die linearen kristallinen Polyphosphate der Erfindung können durch die Formel A beschrieben werden:

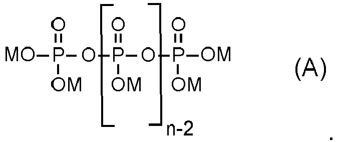

In der Formel A steht n im Mittel (Zahlenmittel) für eine Zahl von 4 bis 2000, häufig 6 bis 1000 oder 8 bis 500, vorzugsweise 10 bis 400 oder 12 bis 300 und insbesondere 15 bis 200. M steht für ein Kation bzw. ein Kationenäquivalent, das vorzugsweise unter Alkalimetall-, Erdalkalimetall- und Ammoniumionen, unter Na⁺, K⁺, NH₄⁺, ½ Ca²⁺ und ½ Mg²⁺, und vorzugsweise unter Na⁺, K⁺, NH₄⁺ und ½ Ca²⁺, ausgewählt ist. Die Ausdrücke "½ Ca²⁺" bzw. "½ Mg²⁺" verdeutlichen hier, dass jedes Ca²⁺ bzw. Mg²⁺, welches in dem Polyphosphat der Formel A enthalten ist, aufgrund seiner zweifach positiven Ladung für zwei Kationen M in der Formel A steht.

Bevorzugt ist das kristalline, anorganische Polyphosphat unter kristallinen, anorganischen Natriumpolyphosphaten, insbesondere linearen Natriumpolyphosphaten der Formel A ausgewählt. Speziell handelt es sich bei dem kristallinen, anorganischen Polyphosphat um ein sogenanntes Maddrell-Salz, das auch als Maddrell'sches Salz bezeichnet wird. Unter Maddrell-Salz, das auch als Maddrell'sches Salz bezeichnet wird, versteht der Fachmann ein hochkondensiertes lineares Natriumpolyphosphat, das typischerweise im Mittel wenigstens 20 Phosphoratome pro Polyphosphat-Anion aufweist.

Die in den erfindungsgemäßen Pads und Folien enthaltenen kristallinen Polyphosphate zeichnen sich durch hohe Reinheiten aus. So enthalten sie nur geringe Mengen an wasserlöslichen Bestandteilen. Als wasserlöslich werden hier solche Bestandteile bezeichnet, die sich in Form eines Pulvers bei 25 °C mit einer Rate von mindestens 50 Gew.-% pro Stunde in Wasser auflösen. Bei solchen wasserlöslichen Bestandteilen handelt es sich typischerweise um Mono-, Pyro- und Triphosphate sowie um cyclische Metaphosphate, wie Tri-, Tetra- oder Hexametaphosphate, die eine hohe Wasserlöslichkeit besitzen. Der Anteil an wasserlöslichen Bestandteilen beträgt in der Regel weniger als 8 Gew.-%, vorzugsweise weniger als 5 Gew.-% und insbesondere weniger als 3 Gew.-% in Bezug auf das Gesamtgewicht des Polyphophats. Der Anteil an Wasser, das als Restfeuchte noch im kristallinen Polyphosphat enthalten sein kann, beträgt üblicherweise weniger als 1 Gew.-%, vorzugsweise weniger als 0,5 Gew.-% und insbesondere weniger als 0,3 Gew.-%, bezogen auf das Gesamtgewicht des Polyphosphats. Der Anteil an Inhaltsstoffen, bei denen es sich nicht um (Poly)Phosphate oder Wasser handelt, liegt in der Regel bei weniger als 0,1 Gew.-%, bevorzugt weniger als 0,05 Gew.-% und insbesondere weniger als 0,01 Gew.-%, in Bezug auf das Gesamtgewicht des Polyphophats.

Die in den erfindungsgemäßen Mitteln enthaltenen kristallinen, anorganischen Polyphosphate liegen in dem Polymermaterial, welches das Flächengebilde bildet, in fein verteilter Form, d. h. in Form von kristallinen Polyphosphat-Partikeln, vor. Dementsprechend bildet das Polymermaterial typischerweise eine kontinuierliche Matrix, welche die darin verteilten Polyphosphat-Partikel umhüllt oder zumindest partiell umgibt.

Die Partikelgröße der kristallinen, anorganischen Polyphosphat-Partikel entspricht in der Regel dem zur Herstellung eingesetzten kristallinen, anorganischen Polyphosphat. Typischerweise weisen die kristallinen Polyphosphat-Partikel Teilchengrößen im Bereich von 0,1 bis 80 µm, insbesondere im Bereich von 0,3 bis 50 µm auf. Die kristallinen Polyphosphat-Partikel weisen üblicherweise einen gewichtsmittleren Teilchendurchmesser im Bereich von 3 bis 50 µm, insbesondere im Bereich von 5 bis 40 µm und speziell 10 bis 30 µm auf. Die hier angegebenen Werte sind die sogenannten d₅₀-Werte der integralen Masseverteilung der Teilchengrößen bzw. der Korngrößen des zur Herstellung eingesetzten kristallinen, anorganischen Polyphosphats.

Die Ermittlung der Teilchengrößenverteilung der kristallinen anorganischen Polyphosphat-Partikel erfolgt typischerweise durch statische Laserlichtstreuung nach Maßgabe der in ISO 13320:2009 beschriebenen Methode. Hieraus lassen sich die Massenanteile der jeweiligen Korngrößen bestimmen. Alternativ können die Massenanteile der jeweiligen Korngrößen bzw. Korngrößenbereiche nach Maßgabe der DIN 66165:2016-08 durch Fraktionierung des Polyphosphats unter Verwendung von mehreren Sieben mittels maschineller Siebung in vorkalibrierten Systemen besimmt werden. Soweit hier und im Folgenden nichts anderes angegeben ist, erfolgt die Bestimmung der Teilchengrößenverteilung von partikulären Substanzen durch statische Laserlichtstreuung nach Maßgabe der in ISO 13320:2009 beschriebenen Methode.

Sofern nichts anderes angegeben ist, sind Prozentangaben im Zusammenhang mit Partikel- bzw. Korngrößen als Angaben in Gew.-% zu verstehen. In diesem Zusammenhang bezeichnet der d₉₀-Wert diejenige Korn- bzw. Partikelgröße, die von 90 Gew.-% der Polyphosphat-Partikel unterschritten wird. Der d₁₀-Wert bezeichnet diejenige Korn-Partikelgröße, die von 10 Gew.-% der Polyphosphat-Partikel unterschritten wird. Der d₅₀-Wert bezeichnet die gewichtsmittlere Korn- bzw. Partikelgröße des Granulats. Bevorzugt weist die Teilchengrößenverteilung des zur Herstellung eingesetzten kristallinen Polyphosphats einen d₉₀-Wert von maximal 70 µm, insbesondere maximal 50 µm und speziell maximal 40 µm auf. Vorzugsweise weist die Teilchengrößenverteilung des zur Herstellung eingesetzten kristallinen Polyphosphats einen d₁₀-Wert von mindestens 0,5 µm, insbesondere mindestens 1 µm und speziell mindestens 2 µm auf.

Die erfindungsgemäßen Mittel enthalten das kristalline anorganische Polyphosphat typischerweise in einer Menge im Bereich von 1 bis 30 Gew.-%, insbesondere in einer Menge im Bereich von 2 bis 20 Gew.-% und speziell in einer Menge im Bereich von 3 bis 15 Gew.-%, bezogen auf das Gesamtgewicht des Mittels.

Bei den organischen Polymermaterialien, welche das Flächengebilde bilden, handelt es sich in der Regel um thermoplastische Polymermaterialien oder um Hydrogele auf Basis organischer Polymere.

Gemäß einer ersten Gruppe von Ausführungsformen der Erfindung handelt es sich bei den Polymermaterialien um thermoplastische Polymermaterialien. Diese bestehen üblicherweise zu wenigstens 50 Gew.-%, bezogen auf die Gesamtmasse des Polymermaterials + Polyphosphat, aus einem oder mehreren organischen, thermoplastischen Polymeren. Insbesondere beträgt der Anteil an organischen, thermoplastischen Polymeren in derartigen thermoplastischen Polymermaterialien wenigstens 60 Gew.-% und speziell wenigstens 65 Gew.-%, bezogen auf die Gesamtmasse des Polymermaterials + Polyphosphat. Häufig beträgt der Anteil an organischen, thermoplastischen Polymeren in derartigen thermoplastischen Polymermaterialien 50 bis 99 Gew.-%, insbesondere wenigstens 60 bis 97 Gew.-% und speziell 65 bis 95 Gew.-%, bezogen auf die Gesamtmasse des Polymermaterials + Polyphosphat. In dieser Gruppe von Ausführungsformen liegt der Gehalt an Polyphosphat in den erfindungsgemäßen Mitteln typischerweise im Bereich von 1 bis 30 Gew.-%, insbesondere im Bereich von 2 bis 20 Gew.-% und speziell im Bereich von 3 bis 15 Gew.-%, bezogen auf die Gesamtmasse des Polymermaterials + Polyphosphat.

Beispiele für geeignete organische, thermoplastische Polymere in thermoplastischen Polymermaterialien sind vor allem Kondensationspolymere wie Polyester, einschließlich aliphatischer Polyester, teilaromatischer Polyester sowie aromatischer Polyester, weiterhin Polyamide, einschließlich aliphatischer Polyamide und teilaromatischer Polyamide, Polyesteramide, einschließlich aliphatischer Polyesteramide und teilaromatischer Polyesteramide, Polycarbonate, Polyestercarbonate aber auch Additionspolymere wie Polystyrole, Polyacrylate, Polyolefine, einschließlich Polyethylen wie HDPE, LDPE, LLDPE, HMW und UHMW, Polypropylene, wie ataktisches, syndiotaktisches oder isotaktisches Polyproplyen, und Polyisoprene, z. B. ataktisches, syndiotaktisches oder isotaktisches Polyisopren, Polyharnstoffe und Polyurethane, einschließlich Polyesterurethane und Polyetherurethane, weiterhin Polysiloxane sowie Blends der vorgenannten Polymere. Bevorzugte thermoplastische Polymere sind unter Polyhydroxyalkanoaten, Polylactiden, aliphatisch-aromatischen Polyestern, aliphatisch-aromatischen Polyamiden, Polyolefinen und/oder deren Gemischen ausgewählt. Insbesondere umfasst das Polymermaterial wenigstens ein Kondensationspolymer, das insbesondere ausgewählt ist unter Polyestern und Polyestercarbonaten und deren Gemischen.

Vorzugsweise umfasst das thermoplastisch verarbeitbare Polymermaterial mindestens ein organisches Polymer, das eine Glasübergangstemperatur oder einen Schmelzpunkt im Bereich von 75 bis 250 °C aufweist. Sofern das Polymer einen Schmelzpunkt hat, also teilkristallin oder kristallin ist, weist es bevorzugt einen Schmelzpunkt im Bereich von 75 bis 250 °C auf. Sofern das Polymer amorph ist, weist es bevorzugt eine Glasübergangstemperatur im Bereich von 75 bis 250 °C auf. Dabei wird der Schmelzpunkt bzw. die Glasübergangstemperatur üblicherweise mittels dynamischer Differenzkalorimetrie (DSC) gemäß DIN EN ISO 11357:2017 bestimmt.

In einer bevorzugten Ausführungsform der Erfindung enthält das thermoplastisch verarbeitbare Polymermaterial als Hauptbestandteil, d. h. zu wenigstens 50 Gew.-%, insbesondere zu wenigstens 60 Gew.-%, speziell zu wenigstens 80 Gew.-% oder zu wenigstens 90 Gew.-%, bezogen auf die darin enthaltenen organischen Polymere, wenigstens ein Polymer, das unter Polyhydroxyalkanoaten, Polylactiden, aliphatisch-aromatischen Polyestern, aliphatisch-aromatischen Polyamiden, Polyolefinen und/oder deren Gemischen ausgewählt ist.

In dieser bevorzugten Ausführungsform der Erfindung enthält das thermoplastisch verarbeitbare Polymermaterial als Hauptbestandteil insbesondere:
i. ein Gemisch aus wenigstens einem Polylactid und wenigstens einem aliphatisch-aromatischen Polyester;
   oder
ii. wenigstens einen Copolyester einer Hydroxybuttersäure mit einer Hydroxyalkansäure, die 6 bis 12 C-Atome aufweist, oder ein Gemisch eines solchen Copolyesters mit Polylactid;
   oder
iii. wenigstens ein Polyolefin, vorzugsweise Polyethylen oder Polypropylen;
   oder
iv. syndiotaktisches, isotaktisches oder ataktisches Polyisopren;
   oder
v. Polysiloxan.

In einer bevorzugten Ausführungsform der Erfindung enthält das thermoplastisch verarbeitbare Polymermaterial als Hauptbestandteil, d. h. zu wenigstens 50 Gew.-%, insbesondere zu wenigstens 60 Gew.-%, speziell zu wenigstens 80 Gew.-% oder zu wenigstens 90 Gew.-%, bezogen auf die darin enthaltenen organischen Polymere, wenigstens ein biologisch abbaubares Polymer.

Unter dem Begriff "biologisch abbaubar" versteht man, dass die in Rede stehende Substanz, hier das Polymer, in dem Test der OECD Richtlinie 301B von 1992 (Messung der CO₂ Entwicklung bei Kompostierung in einem mineralischen Schlamm und Vergleich mit der theoretisch maximal möglichen CO₂ Entwicklung) nach sieben Tagen bei 25 °C zu wenigstens 5 % abgebaut ist.

Beispiele für bevorzugte, biologisch abbaubare Polymere sind Polyhydroxyalkanoate, Polylactide, aliphatisch-aromatische Polyester, aliphatisch-aromatische Polyamide, aliphatisch-aromatische Polyesteramide und deren Gemische. Insbesondere ist das biologisch abbaubare Polymer unter aliphatisch-aromatischen Polyestern und deren Gemischen mit Polyhydroxyalkanoaten und/oder Polylactiden ausgewählt.

Teilaromatische Polyester werden auch als aliphatisch-aromatische Polyester bezeichnet, d. h. Polyester auf Basis von aromatischen Dicarbonsäuren und aliphatischen Dihydroxyverbindungen sowie Polyestern auf Basis von Gemischen aromatischer Dicarbonsäuren mit aliphatischen Dicarbonsäuren und aliphatischen Dihydroxyverbindungen. Bei den aliphatisch-aromatischen Polyestern handelt es sich insbesondere um Polyester auf Basis von Gemischen aliphatischer Dicarbonsäuren mit aromatischen Dicarbonsäuren und aliphatischen Dihydroxyverbindungen.

Aliphatische Dicarbonsäuren sind beispielsweise Oxalsäure, Malonsäure, Bernsteinsäure, 2-Methylbernsteinsäure, Glutarsäure, 2-Methylglutarsäure, 3-Methylglutarsäure, α-Ketoglutarsäure, Adipinsäure, Pimelinsäure, Azelainsäure, Sebacinsäure, Brassylsäure, Fumarsäure, 2,2-Dimethylglutarsäure, Suberinsäure (Korksäure), Diglykolsäure, Oxalessigsäure, Glutaminsäure, Asparaginsäure, Itaconsäure, Maleinsäure und deren Gemische. Bevorzugte aliphatische Dicarbonsäuren sind ausgewählt unter Bernsteinsäure, Adipinsäure, Azelainsäure, Sebacinsäure, Brassylsäure und deren Mischungen.

Bei der aromatischen Dicarbonsäure handelt es sich insbesondere um Terephthalsäure.

Bei den aliphatischen Diolen handelt es sich insbesondere um verzweigte oder lineare Alkandiole mit 2 bis 12 Kohlenstoffatomen, insbesondere 4 bis 6 Kohlenstoffatomen. Beispiele geeigneter Alkandiole sind Ethylenglykol, 1,2-Propandiol, 1,3-Propandiol, 1,2-Butandiol, 1,4-Butandiol, 1,5-Pentandiol, 2,4-Dimethyl-2-ethylhexan-1,3-diol, 2,2-Dimethyl-1,3-propandiol, 2-Ethyl-2-butyl-1,3-propandiol, 2-Ethyl-2-isobutyl-1,3-propandiol, 2,2,4-Trimethyl-1,6-hexandiol, insbesondere Ethylenglykol, 1,3-Propandiol, 1,4-Butandiol und 2,2-Dimethyl-1,3-propandiol (Neopentylglykol).

Besonders bevorzugt ist der aliphatisch-aromatische Polyester ausgewählt unter Polybutylenazealat-co-Butylenterephthalat (PBAzeT), Polybutylenbrassylat-co-butylenterephthalat (PBBrasT), Polybutylenadipatterephthalat (PBAT), Polybutylensebacatterephthalat (PBSeT) und Polybutylensuccinatterephthalat (PBST) und deren Gemischen.

Zu den Polylactiden zählen Polymilchsäure sowie Polymilchsäure-Copolymere wie Polylactid-co-Polyglykolsäure (PLGA). Zu den Polyhydroxyalkanoaten zählen insbesondere Homo- und Copolyesters von 3- oder 4-Hydroxyalkansäuren mit 4 bis 12-C-Atomen, beispielsweise Polyhydroxybutyrate wie Poly-4-hydroxybutyrate und Poly-3-hydroxybutyrate, Polyhydroxyvalerate, wie Homo- und Copolyester der 3-Hydroxyvalersäure, Polyhydroxyhexanoate wie Homo- und Copolymere der 3-Hydroxyhexansäure. Hierunter besonders bevorzugt sind insbesondere Copolyester der vorgenannten Hydroxybuttersäuren mit längerkettigen Hydroxyalkansäuren, die vorzugsweise 6 bis 12 C-Atome aufweisen, beispielsweise Copolyester einer Hydroxybuttersäure mit einer Hydroxyvalersäure, z. B. (P(3HB)-co-P(3HV)), sowie Copolyester einer Hydroxybuttersäure mit einer Hydroxyhexansäure.

Teilaromatische Polyamide werden auch als aliphatisch-aromatische Polyamide bezeichnet, d. h. Polyamide auf Basis von aromatischen Dicarbonsäuren und aliphatischen Diaminoverbindungen sowie Polyamide auf Basis von Kombinationen aromatischer Dicarbonsäuren mit aliphatischen Dicarbonsäuren und aliphatischen Diaminoverbindungen.

Teilaromatische Polyesteramide werden auch als aliphatisch-aromatische Polyesteramide bezeichnet, d. h. Polyesteramide auf Basis von aromatischen Dicarbonsäuren und Kombinationen aliphatischer Dihydroxyverbindungen und aliphatischer Diaminoverbindungen sowie Polyesteramide auf Basis von Kombinationen aromatischer Dicarbonsäuren mit aliphatischen Dicarbonsäuren und Kombinationen aliphatischer Dihydroxyverbindungen und aliphatischer Diaminoverbindungen.

Bei den aliphatisch-aromatischen Polyestern handelt es sich insbesondere um Polyester auf Basis von Gemischen aliphatischer Dicarbonsäuren mit aromatischen Dicarbonsäuren und aliphatischen Dihydroxyverbindungen.

Bevorzugt umfassen die thermoplastisch verarbeitbaren, organischen Polymermaterialien, bezogen auf die darin enthaltenen organischen Polymere, wenigstens einen aliphatisch-aromatischen Polyester, insbesondere wenigstens einen der als besonders bevorzugt genannten aliphatisch-aromatischen Polyester. Insbesondere ist der wenigstens eine aliphatisch-aromatische Polyester Hauptbestandteil und macht wenigstens 50 Gew.-%, insbesondere wenigstens 60 Gew.-%, bezogen auf die im Polymermaterial enthaltenen organischen Polymere aus.

Insbesondere umfassen die thermoplastisch verarbeitbaren, organischen Polymermaterialien als Hauptbestandteil, d. h. zu wenigstens 50 Gew.-%, vorzugsweise zu wenigstens 60 Gew.-%, insbesondere zu wenigstens 80 Gew.-% oder zu wenigstens 90 Gew.-%, bezogen auf die darin enthaltenen organischen Polymere, ein Gemisch aus wenigstens einem Polylactid und wenigstens einem aliphatisch-aromatischen Polyester, insbesondere wenigstens einen der als besonders bevorzugt genannten aliphatisch-aromatischen Polyester.

In einer bevorzugten Gruppe von Ausführungsformen umfassen die thermoplastisch verarbeitbaren, organischen Polymermaterialien als Hauptbestandteil, d. h. zu wenigstens 50 Gew.-%, vorzugsweise zu wenigstens 60 Gew.-%, insbesondere zu wenigstens 80 Gew.-% oder zu wenigstens 90 Gew.-%, bezogen auf die darin enthaltenen organischen Polymere, ein Gemisch enthaltend:
- 1 bis 40 Gew.-%, insbesondere 2 bis 30 Gew.-% wenigstens eines Polylactids und
- 60 bis 99 Gew.-%, insbesondere 70 bis 98 Gew.-% wenigstens eines aliphatisch-aromatischen Polyesters, insbesondere wenigstens eines der als besonders bevorzugt genannten aliphatisch-aromatischen Polyester,
wobei die Angaben in Gew.-% auf die Gesamtmasse aus Polylactid und aliphatisch-aromatischen Polyester bezogen sind.

In einer weiteren bevorzugten Gruppe von Ausführungsformen umfassen die thermoplastisch verarbeitbaren, organischen Polymermaterialien als Hauptbestandteil, d. h. zu wenigstens 50 Gew.-%, vorzugsweise zu wenigstens 60 Gew.-%, insbesondere zu wenigstens 80 Gew.-% oder zu wenigstens 90 Gew.-%, bezogen auf die darin enthaltenen organischen Polymere, wenigstens einen Copolyester einer Hydroxybuttersäure mit einer Hydroxyalkansäure, die 6 bis 12 C-Atome aufweist, beispielsweise wenigstens einen Copolyester einer Hydroxybuttersäure mit einer Hydroxyvalersäure, z. B. (P(3HB)-co-P(3HV)), und/oder einen Copolyester einer Hydroxybuttersäure mit einer Hydroxyhexanäure, oder ein Gemisch eines oder mehrerer solcher Copolyester mit wenigstens einem Polylactid.

Neben dem organischen Polymeren kann das Polymermaterial auch typische Verarbeitungshilfsmittel enthalten.

Zu den Verarbeitungshilfsmitteln zählen Dispergiermittel, Gleitmittel, Hydrophobierungsmittel, Phasenvermittler, Vernetzungsmittel, Mischungsverbesserer, Weichmacher, Katalysatoren und Antioxidantien sowie Kettenverlängerer. Bevorzugt sind die Verarbeitungshilfsmittel physiologisch verträglich und/oder haben eine Zulassung für die Verwendung in Medizinprodukten.

Neben dem Polymermaterial und dem Polyphosphat können die Mittel auf Basis thermoplastischer Polymermaterialien gegebenenfalls Füllstoffe enthalten, die von dem anorganischen Polyphosphat verschieden sind. Zu den weiteren Füllstoffen zählen insbesondere anorganische Füllstoffe, wie sie üblicherweise in thermoplastischen Polymermaterialen Anwendung finden. Bevorzugt sind die weiteren Füllstoffe physiologisch verträglich und/oder haben eine Zulassung für die Verwendung in Medizinprodukten. Beispiele für geeignete Füllstoffe sind Kreide, Kieselgel und Tonmineralien wie Talkum, oder Bentonit und deren Gemische.

Die weiteren Füllstoffe weisen üblicherweise einen gewichtsmittleren Teilchendurchmesser (d₅₀-Wert) im Bereich von 3 bis 50 µm, insbesondere im Bereich von 5 bis 40 µm und speziell 10 bis 30 µm auf, bestimmt durch statische Laserlichtstreuung nach Maßgabe der in ISO 13320:2009 beschriebenen Methode oder nach Maßgabe der DIN 66165:2016-08 durch Fraktionierung des Füllstoffs unter Verwendung von mehreren Sieben mittels maschineller Siebung in vorkalibrierten Systemen.

Der Anteil an Verarbeitungshilfsmitteln liegt typischerweise im Bereich von 0 bis 5 Gew.-%. Der Anteil weiterer Füllstoffe liegt typischerweise im Bereich von 0 bis 25 Gew.-% oder im Bereich von 1 bis 25 Gew.-%, bezogen auf die Gesamtmasse des Polymermaterials + Polyphosphat + weiterem Füllstoff. Die Gesamtmasse an Polyphosphat und davon verschiedenen Füllstoffen liegt typischerweise im Bereich von 1 bis 50 Gew.-%, insbesondere im Bereich von 2 bis 40 Gew.-%, und speziell im Bereich von 3 bis 35 Gew.-%, bezogen auf das Polymermaterial + Polyphosphat + weiterem Füllstoff.

In einer zweiten Gruppe von Ausführungsformen der Erfindung handelt es sich bei dem Polymermaterial um ein Hydrogel auf Basis organischer Polymere. Hierbei handelt es sich um Wasser enthaltende Gele auf der Basis vernetzter, in Wasser quellbarer, aber gleichzeitig wasserunlöslicher Polymere, die auch als Hydrogelbildner bezeichnet werden (siehe Römpp Chemie-Lexikon, 10. Auflage, Georg Thieme Verlag 1997, S. 1835 und dort zitierte Literatur).

Geeignete Hydrogelbildner sind vernetzte Poly(meth)acrylsäuren, vernetzte Polyvinylalkohole, vernetzte Polyvinylpyrrolidone, vernetzte Polyalkylenether und vorzugweise vernetzte Polysaccharide wie Carragene, Gellane Agarose, Xanthan, Stärke, Chitosan, Carboxymethylcellulose, Pektine und Alginate. Übersichten über Hydrogele auf Basis vernetzter Polysaccharide findet man von J. T. Oliveira et al. in "Natural-Based Polymers for Biomedical Applications", Kapitel 18: "Hydrogels from polysaccharidebased materials: Fundamentals and applications in regenerative medicine", Woodhead Publishing in Biomaterials 2008, S. 485-514, https://doi.org/10.1533/9781845694814.4.485 und von D. Pasqui et al., Polymers 2012, 4, 1517-1534; doi:10.3390/polym4031517. Die Vernetzung des Polysaccharids kann durch kovalente Vernetzungsmittel wie auch durch koordinative Vernetzungsmittel, z. B. durch Salze mehrwertiger Metallionen, bewirkt werden. Hierzu zählen insbesondere Salze der Erdalkalimetalle, insbesondere Calciumsalze wie Calciumchlorid, Calciumcarbonat oder Calciumsulfat, sowie Zinksalze wie Zinkchlorid oder Zinksulfat.

Erfindungsgemäße bevorzugte Hydrogele enthalten als Hydrogelbildner wenigstens ein vernetztes Polysaccharid, insbesondere wenigstens ein vernetztes carboxyliertes Polysaccharid, wie Carboxymethylcellulose, Pektine und Alginate, und speziell ein vernetztes Alginat. Die Vernetzung des carboxylierten Polysaccharids erfolgt insbesondere durch koordinative Vernetzungsmittel. Insbesondere wird die Vernetzung durch Salze mehrwertiger Metallionen bewirkt. Hierzu zählen insbesondere die vorgenannten Salze der Erdalkalimetalle sowie die vorgenannten Zinksalze.

In dieser zweiten Gruppe von Ausführungsformen enthält das erfindungsgemäße Mittel typischerweise den Hydrogelbildner in einer Menge im Bereich von 1 bis 20 Gew.-%, insbesondere in einer Menge im Bereich von 2 bis 15 Gew.-%, bezogen auf die Gesamtmasse des Hydrogels + Polyphosphat. Neben dem Hydrogelbildner enthält das Hydrogel Wasser sowie das kristalline, anorganische Polyphosphat. Der Wassergehalt im Hydrogel liegt vorzugsweise im Bereich von 20 bis 80 Gew.-%, bezogen auf die Gesamtmasse an Hydrogelbildner, Wasser und Polyphosphat. In dieser Gruppe von Ausführungsformen liegt der Gehalt an Polyphosphat in den erfindungsgemäßen Mitteln typischerweise im Bereich von 1 bis 30 Gew.-%, insbesondere im Bereich von 2 bis 20 Gew.-%, und speziell im Bereich von 3 bis 15 Gew.-%, bezogen auf die Gesamtmasse des Hydrogelbildners, Wasser und Polyphosphat.

Neben den vorgenannten Bestandteilen kann das erfindungsgemäße Mittel auf Basis des Hydrogels weitere Bestandteile enthalten, die im Hinblick auf seine Herstellung erforderlich sind. Hierzu zählen insbesondere Puffer und Verzögerer, z. B. Alkalimetallpyrophosphate und Reaktionsprodukte, die bei der Umsetzung des unvernetzten Hydrogelbildners mit dem eingesetzten Vernetzungsmittel gebildet werden, beispielsweise anorganische Säuren und deren Salze.

Die erfindungsgemäßen Mittel können außerdem ein oder mehrere weitere, von den kristallinen, anorganischen Polyphosphaten verschiedene Agentien enthalten, die bekanntermaßen eine blutstillende Wirkung aufweisen. Eine Übersicht findet man beispielsweise in G. Lawton et al., JR. Army Med. Corps 155(4) (2009) 309-314 mit weiteren Nachweisen. Vorzugsweise handelt es sich um ein organisches Agens, beispielsweise um Chitosan, dessen Derivate, Thrombin, Fibrin oder Fibrinogen. Der Anteil derartiger weiterer Agentien mit blutstillender Wirkung wird in der Regel 10 Gew.-%, bezogen auf das Gewicht der erfindungsgemäßen Mittel nicht überschreiten und liegt vorzugsweise bei maximal 5 Gew.-%, bezogen auf das Gewicht der erfindungsgemäßen Mittel. Insbesondere enthalten die erfindungsgemäßen Mittel keine weiteren anorganischen Agentien mit blutstillender Wirkung, die von kristallinem anorganischen Polyphosphat verschieden sind.

In einer bevorzugten Ausführungsform der Erfindung werden die erfindungsgemäßen Mittel in steriler Form eingesetzt. Demgemäß betrifft die Erfindung auch erfindungsgemäße Mittel, die steril sind sowie erfindungsgemäße Mittel in steriler Form, die in einer Verpackung eingeschlossen sind, so dass sie auch über längere Lagerperioden ihre Sterilität nicht verlieren.

Eine besonders bevorzugte Gruppe von Ausführungsformen der Erfindung betrifft Folien aus den zuvor beschriebenen thermoplastischen Polymermaterialien, welche wenigstens ein kristallines, anorganisches Polyphosphat enthalten.

Bevorzugte Folien weisen eine Dicke im Bereich von 10 bis 250 µm, insbesondere im Bereich von 20 bis 200 µm auf. Insbesondere enthalten sie als kristallines, anorganisches Polyphosphat ein Polyphosphat, das einen gewichtsmittleren Teilchendurchmesser d₅₀ im Bereich von 3 bis 50 µm, insbesondere im Bereich von 5 bis 40 µm und speziell 10 bis 30 µm aufweist. Insbesondere ist der Teilchendurchmesser so abgestimmt, dass das Verhältnis von Foliendicke zu gewichtsmittlerem Teilchendurchmesser des partikulären Polyphosphats im Bereich von 0,5 : 1 bis 5 : 1 liegt.

Hinsichtlich der zuvor im Zusammenhang mit thermoplastischen Polymermaterialien, Polyphosphaten, weiteren Bestandteilen und Mengenanteilen erwähnten Bevorzugungen gilt sinngemäß Gleiches für die in den Folien enthaltenen Bestandteile.

Insbesondere bestehen die in den Folien enthaltenen thermoplastisch verarbeitbaren, organischen Polymermaterialien zu wenigstens 50 Gew.-%, bezogen auf die Gesamtmasse des Folienmaterials, aus einem oder mehreren organischen, thermoplastischen Polymeren. Insbesondere beträgt der Anteil an organischen, thermoplastischen Polymeren in derartigen thermoplastischen Polymermaterialien wenigstens 60 Gew.-% und speziell wenigstens 65 Gew.-%, bezogen auf die Gesamtmasse des Folienmaterials. Häufig beträgt der Anteil an organischen Polymeren in derartigen thermoplastischen Polymermaterialien 50 bis 99 Gew.-%, insbesondere wenigstens 60 bis 97 Gew.-% und speziell 65 bis 95 Gew.-%, bezogen auf die Gesamtmasse des Folienmaterials. In dieser Gruppe von Ausführungsformen liegt der Gehalt an Polyphosphat in den Folienmaterialien typischerweise im Bereich von 1 bis 30 Gew.-%, insbesondere im Bereich von 2 bis 20 Gew.-%, und speziell im Bereich von 3 bis 15 Gew.-%, bezogen auf die Gesamtmasse des Polymermaterials + Polyphosphat.

In einer bevorzugten Gruppe von Ausführungsformen umfassen die in den Folien enthaltenen thermoplastisch verarbeitbaren, organischen Polymermaterialien als Hauptbestandteil, d. h. zu wenigstens 50 Gew.-%, vorzugsweise zu wenigstens 60 Gew.-%, insbesondere zu wenigstens 80 Gew.-% oder zu wenigstens 90 Gew.-%, bezogen auf die darin enthaltenen organischen Polymere, ein Gemisch, enthaltend:
- 1 bis 40 Gew.-%, insbesondere 2 bis 30 Gew.-% wenigstens eines Polylactids
   und
- 60 bis 99 Gew.-%, insbesondere 70 bis 98 Gew.-% wenigstens eines aliphatisch-aromatischen Polyesters, insbesondere wenigstens eines der als besonders bevorzugt genannten aliphatisch-aromatischen Polyester,
wobei die Angaben in Gew.-% auf die Gesamtmasse aus Polylactid und aliphatisch-aromatischen Polyester bezogen sind.

In einer weiteren bevorzugten Gruppe von Ausführungsformen umfassen die in den Folien enthaltenen thermoplastisch verarbeitbaren, organischen Polymermaterialien als Hauptbestandteil, d. h. zu wenigstens 50 Gew.-%, vorzugsweise zu wenigstens 60 Gew.-%, insbesondere zu wenigstens 80 Gew.-% oder zu wenigstens 90 Gew.-%, bezogen auf die darin enthaltenen organischen Polymere, wenigstens einen Copolyester einer Hydroxybuttersäure mit einer Hydroxyalkansäure, die 6 bis 12 C-Atome aufweist, beispielsweise wenigstens einen Copolyester einer Hydroxybuttersäure mit einer Hydroxyvalersäure, z. B. (P(3HB)-co-P(3HV)), und/oder einen Copolyester einer Hydroxybuttersäure mit einer Hydroxyhexansäure, oder ein Gemisch eines oder mehrerer solcher Copolyester mit wenigstens einem Polylactid.

Gegebenenfalls enthalten diese Folien wenigstens einen weiteren anorganischen Füllstoff. Insbesondere handelt es sich bei dem weiteren Füllstoff um Kreide, Talkum und/oder um Mischungen davon. Der Anteil weiterer Füllstoffe liegt, sofern vorhanden, typischerweise im Bereich von 1 bis 25 Gew.-%, bezogen auf die Gesamtmasse des Folienmaterials. Die Gesamtmasse an Polyphosphat und den gegebenenfalls vorhandenen, davon verschiedenen Füllstoffen wird vorzugsweise 50 Gew.-%, insbesondere 40 Gew.-% und speziell 35 Gew.-% nicht überschreiten und liegt typischerweise im Bereich von 1 bis 50 Gew.-%, insbesondere im Bereich von 2 bis 40 Gew.-%, und speziell im Bereich von 3 bis 35 Gew.-%, bezogen auf das Folienmaterial.

Eine weitere besonders bevorzugte Gruppe von Ausführungsformen der Erfindung betrifft Pads aus den zuvor beschriebenen Hydrogelen, welche wenigstens ein kristallines anorganisches Polyphosphat enthalten.

Bevorzugte Pads weisen eine Dicke im Bereich von 4 bis 20 mm und insbesondere im Bereich von 10 bis 17 mm auf. Insbesondere enthalten sie als kristallines, anorganisches Polyphosphat ein Polyphosphat, das einen gewichtsmittleren Teilchendurchmesser d₅₀ im Bereich von 5 bis 40 µm, insbesondere im Bereich von 10 bis 30 µm aufweist.

Hinsichtlich der zuvor im Zusammenhang mit Hydrogelbildnern, Polyphosphaten, wieteren Bestandteilen und Mengenanteilen erwähnten Bevorzugungen gilt sinngemäß Gleiches für die in den Pads enthaltenen Bestandteile.

Insbesondere enthalten die Pads als Hydrogelbildner wenigstens ein vernetztes Polysaccharid, insbesondere wenigstens ein vernetztes carboxyliertes Polysaccharid und speziell ein vernetztes Alginat. Die Vernetzung des carboxylierten Polysaccharids erfolgt insbesondere durch koordinative Vernetzungsmittel. Insbesondere wird die Vernetzung durch Salze mehrwertiger Metallionen bewirkt. Hierzu zählen insbesondere die vorgenannten Salze der Erdalkalimetalle sowie die vorgenannten Zinksalze.

Insbesondere enthält das Pad den Hydrogelbildner in einer Menge im Bereich von 2 bis 15 Gew.-%, bezogen auf die Gesamtmasse des Pads. Neben dem Hydrogelbildner enthält das Pad Wasser sowie das kristalline, anorganische Polyphosphat. Der Wassergehalt im Pad liegt vorzugsweise im Bereich von 30 bis 90 Gew.-%, bezogen auf die Gesamtmasse des Pads. Insbesondere liegt der Gehalt an Polyphosphat in dem Pad typischerweise im Bereich von 1 bis 30 Gew.-%, insbesondere im Bereich von 2 bis 20 Gew.-%, und speziell im Bereich von 3 bis 15 Gew.-%, bezogen auf die Gesamtmasse des Pads.

Die Herstellung der erfindungsgemäßen Mittel kann in an sich bekannter Weise in Analogie zur Herstellung bekannter Flächengebilde aus Polymermaterialien erfolgen. Die Herstellung umfasst typischerweise das Einarbeiten des Polyphosphats und gegebenenfalls weiterer blutstillender Agenzien in das organische Polymermaterial. Verfahren hierzu sind dem Fachmann geläufig.

Erfindungsgemäße Mittel auf Basis thermoplastischer Polymermaterialien, wie beispielsweise Folien, lassen sich in einfacher Weise dadurch herstellen, dass man das gewünschte thermoplastische Polymere mit dem anorganischen Polyphosphat sowie gegebenenfalls mit Verarbeitungshilfsmitteln und/oder weiteren Füllstoffen zu einem Compound verarbeitet und aus diesem Compound in an sich bekannter Weise, z. B. durch Extrusion, ein Flächengebilde, z. B. eine Folie, herstellt.

Erfindungsgemäße Mittel auf Basis von Hydrogelen, wie beispielsweise Pads, lassen sich in einfacher Weise dadurch herstellen, dass man in einer Lösung des unvernetzten Hydrogelbildners in Wasser das gewünschte anorganische Polyphosphat sowie gegebenenfalls einen oder mehrere weitere Füllstoffe suspendiert, eine Vernetzung des Hydrogelbildners bewirkt und während des Vernetzungsvorgangs ein Flächengebilde formt, beispielsweise durch Formgießen oder durch Extrusion.

Zur Behandlung einer blutenden Wunde wird das erfindungsgemäße Mittel im Wundbereich in der Regel so appliziert, dass die Wunde bzw. der Wundbereich wenigstens teilweise, vorzugsweise vollständig oder zum größten Teil mit dem Mittel bedeckt wird. Nach der Applikation befindet sich das flächige Mittel in unmittelbarer Nähe zum bzw. im Kontakt mit dem Wundgewebe und hat Kontakt zu Blut. Aufgrund seiner Flexibilität ist das Mittel in der Lage, sich der Form der Wunde in gewissem Maß anzupassen und die Wundflächen zumindest zum Teil zu bedecken bzw. weist bereits vor der Applikation in etwa die Form der Wunde auf. Der Fachmann ist daher problemlos in der Lage, für eine verschiedene Wunden oder Blutungsquellen das jeweils beste erfindungsgemäße Mittel zur blutstillenden Behandlung auszuwählen und seine Form dem Wundbereich anzupassen, beispielsweise durch Zuschneiden auf die erforderliche Größe. Aufgrund ihrer flächigen Struktur lassen sich die erfindungsgemäßen Mittel leicht auf der blutenden Wunde applizieren.

Aufgrund ihrer blutstillenden Wirkung eignen sich die erfindungsgemäßen Mittel in besonderer Weise zur Behandlung blutender Wunden. Hierbei kann es sich um oberflächliche Hautverletzungen handeln, aber auch um Verletzungen des Körpergewebes, insbesondere Schädigungen des Gewebes innerer Organe, wie sie bei chirurgischen Eingriffen auftreten und die oftmals einen diffusen, gegebenenfalls auch starken Blutfluss zur Folge haben. Die Gefahr eines Ausspülens des Mittels aus dem Wundbereich oder ein Eindringen des Mittels in die Blutbahn ist aufgrund der flächigen Natur ausgeschlossen. Zudem lässt sich durch Ausüben von Druck auf der blutenden Wunde das applizierte Mittel direkt in den Blutfluss einbringen, so dass die Gerinnung effizienter eintritt.

Dementsprechend eignen sich die erfindungsgemäßen Mittel insbesondere zur Verwendung bei chirurgischen Eingriffen, insbesondere bei Operationen an inneren Organen.

Darüber hinaus kann das Mittel im gesamten Anwendungsbereich der Notfallmedizin eingesetzt werden.

Die erfindungsgemäßen Mittel werden durch die nachfolgenden Abbildungen und Beispiele näher erläutert.

Einsatzstoffe:

| | |
|---|---|
| PBAT: | Polybutylenadipatterephthalat, Ecoflex^{®} der Fa. BASF SE, Ludwigshafen; |
| PBST: | Polybutylen-succinat-terephthalat der Fa. IRe Chemicals Ltd., Süd Korea; |
| PLA: | Polymilchsäure PLA 2003D der Fa. Nature Works; |
| Kreide: | Handelsübliches Kreidepulver mit einem d₅₀-Wert von 1 µm, Produkt der Fa. Omya GmbH, Köln; |
| Talkum: | Handelsübliches Talkum mit einem d₅₀-Wert von 2,2 - 15 µm; |
| Maddrell-Salz: | hochmolekulares kristallines Natriumpolyphosphat in Form eines Pulvers mit einem d₅₀-Wert von 15 µm, einem Anteil löslicher Bestandteile von < 3 Gew.-%, einem Kristallinitätsgrad von > 95 %, einer mittleren Anzahl von P-Atomen pro Polyphosphatanion von 44 und einem Gehalt an Phosphat von 70 Gew.-%, gerechnet als P₂O₅; |
| Natriumalginat: | Hewigum Na 1 der Fa. Hewico Produktions u. Handels GmbH mit einem Trocknungsverlust < 15 Gew.-% und einer Partikelgröße von 177 µm, dessen 1 gew.-%ige Lösung in Wasser bei 22 °C eine Viskosität von 700 mPas aufweist; |
| CaSO₄: | Calciumsulfat-Dihydrat-Pulver pa mit einem d₅₀-Wert von 10 µm (Luxopharm^{®}F211 der Fa. SRL Pharma, Ludiwgshafen); |
| TNPP: | Tetranatriumpyrophosphat E450 (iii), BK Giulini, Ladenburg. |

### Allgemeine Herstellungsvorschrift für Folien

Bitte Herstellungsvorschrift für Extrusion mit Prozessparametern angeben, z. B.:
In einem Extruder Typ Fa. TSA EMP 26-40 mit einem D/L-Verhältnis von 26 : 40, der bei einer Schneckendrehzahl von 200 upm, einem Druck vor Düse von 14 bis 15 bar und dem in Tabelle 1a angegebenen Temperaturprofil betrieben wurde, stellte man einen Compound aus den in Tabelle 1 angegebenen Einsatzsstoffen her.

Der jeweilige Compound wurde in einem Folienextruder Typ ZSE 40 der Fa. Leistritz mit einem D/L-Verhältnis von 26 : 40, der bei einer Schneckendrehzahl von 180 upm, einem Druck vor Düse von 11 bis 12 bar betrieben wurde, zu einer Folie mit einer mittleren Dicke von 15 µm verarbeitet.

**Tabelle 1:**

| | Folie 1 | Folie 2 | Folie 3 | Folie 4 | Folie 5 | Folie 5 |
|---|---|---|---|---|---|---|
| Einsatzstoff | | | | | | |
| PBAT [Gew.-%] | 63,0 | 63,0 | 63,0 | 63,0 | 63,0 | 0 |
| PBST [Gew.-%] | 0 | 0 | 0 | 0 | 0 | 63,0 |
| PLA [Gew.-%] | 10,0 | 10,0 | 10,0 | 11,0 | 15,0 | 10,0 |
| Verarbeitungshilfsmittel 1 [Gew.-%] | 1,0 | 1,0 | 1,0 | 1,0 | 1,0 | 1,0 |
| Verarbeitungshilfsmittel 2 [Gew.-%] | 0,5 | 0,5 | 0,5 | 0,5 | 0,5 | 0,5 |
| Kreide [Gew.-%] | 12,5 | 15,5 | 10,5 | 12,5 | 10,5 | 12,5 |
| Talkum [Gew.-%] | 8,0 | 0 | 0 | 4,0 | 0 | 8,0 |
| Maddrell-Salz [Gew.-%] | 5,0 | 10,0 | 15,0 | 8,0 | 10,0 | 5,0 |

**Tabelle 1a**

| Zone | 1 | 2 | 3 | 4 | 5 | 6 | 7 | 8 Düse |
|---|---|---|---|---|---|---|---|---|
| Soll | 140 | 150 | 155 | 160 | 170 | 175 | 170 | 160 |
| Ist | 140 | 150 | 160 | 170 | 170 | 175 | 171 | 162 |

### Allgemeine Herstellungsvorschrift für Pads aus Hydrogelen

In einem Becherglas wurde Natriumalginat und Tetranatriumpyrophospat in den in Tabelle 2 angegebenen Mengen in Wasser gelöst. Hierzu gab man unter Rühren sukzessive festes Maddrell-Salz und Calciumsulfat-Dihydrat-Pulver, Tetranatriumpyrophosphat in den in Tabelle 2 angegebenen Mengen und rührte danach für weitere 40 sec oder 90 sec mit der in Tabelle 2 angegebenen Umdrehungszahl. Je etwa 75 ml der so erhaltenen Suspension goss man in Petrischalen mit einem Durchmesser von 8,5 cm und beließ die Schalen 1 h bei 23 °C. Auf diese Weise erhielt man Pads mit einer Dicke von 15 mm, einem Gehalt an Maddrell-Salz von 9 Gew.-% und einem Wassergehalt von etwa 85 Gew.-%.

**Tabelle 2:**

| | Versuch 1 | Versuch 2 | Versuch 3 |
|---|---|---|---|
| Einsatzstoff | | | |
| Alginat [g] | 7,2 | 7,2 | 6,8 |
| CaSO₄ [g] | 5,2 | 5,2 | 4,9 |
| TNPP [g] | 1,0 | 1,5 | 1,6 |
| Maddrell-Salz [g] | 20 | 20 | 20 |
| Entionisiertes Wasser [g] | 188 | 188 | 188 |
| | | | |

| Umsetzungsparameter | | | |
|---|---|---|---|
| Rührgeschwindigkeit [upm] | 1000 | 1000 | 1000 |
| Rührzeit [sec] | 90 | 40 | 40 |
| | | | |
| Produkteigenschaften | flexibel | flexibel | flexibel |
| Wassergehalt [Gew.-%] | 84,9 % | 84,72 % | 84,95 % |
| Gehalt an Maddrell-Salz [Gew.-%] | 9 % | 9 % | 9% |

Die folgenden Untersuchungen wurden zur Ermittlung der blutstillenden Wirkung der erfindungsgemäßen Flächengebilde im Vergleich zu einer kommerziell verfügbaren Wundauflage durchgeführt.

Als Wundauflagen wurden die erfindungsgemäße Folie 3 gemäß Tabelle 1, im Folgenden als PG bezeichnet sowie zu Vergleichszwecken die kommerzielle Kaolinhaltige, textile Wundauflage QuickClot Combat Gauze^{®} (Z-Medica), im Folgenden CG eingesetzt.

26 Hausschweine (79±2,4 kg) wurden in zwei Gruppen eingeteilt, die entweder mit der Folie PG (n=14) oder mit der Wundauflage CG (n=12) behandelt wurden. Nach der Basismessung der systemischen Hämodynamik und der lokalen Transitzeit-Messung (Local Transit Time Flow Measurement TTFM) an der Oberschenkelarterie wurde eine standardisierte komplexe Leistenverletzung mit 4,7 mm Punktion der Oberschenkelarterie proximal zur Strömungssonde durchgeführt. Unkontrollierte Blutungen wurden ermöglicht, um einen arteriellen systolischen Solldruck von unter 60 mmHg als Indikator für einen schweren Schock zu erreichen. Alle Wundauflagen wurden 5 Minuten lang unter Anwendung von 200 mmHg Dauerdruck aufgetragen. Nach 5 Minuten wurde der Druck abgebaut, und die Hämostase wurde wiederhergestellt. Die hämodynamischen Ausgangswerte wurden durch Volumensubstitution wiederhergestellt und Katecholamine sowie hämatologische Parameter wurden für zwei Stunden aufgezeichnet. Response-variable Daten wurden als Ausfallzeitverteilung dargestellt, wobei die Zeit bis zum Ereignis als die verstrichene Versuchszeit bis zum Wegfall der Hämostase oder bis zum Versuchsende definiert wurde. Die Daten wurden mit der Kaplan-Meier-Methode und mit Hilfe mehrerer Cox-Regressionen analysiert. Unabhängige Variablen umfassten direkt gemessene hämodynamische Variablen sowie die Differenz zwischen den Werten zur Ereigniszeit, die als Differenz zur Basislinie und zur Schockinduktion berechnet wurden.

Bei allen 26/26 Tieren wurde während der Wiederbelebung nach dem Schockereignis mittels der Wundauflage eine Hämostase erzielt. Nachblutungen traten bei 10/26 der behandelten Schweine auf. Hierbei hatten 3/12 (25 %) der mit CG und 7/14 (50 %) der mit PG behandelten Tiere Nachblutungen (p=0,19). Die verstrichene Zeit bis zum Wiederbluten war zwischen den Gruppen nicht signifikant unterschiedlich (Median: 45 min PG vs. 50 min CG, p<0,983). Es gab keinen statistisch signifikanten Unterschied in den femoralen arteriellen Blutflüssen zwischen den beiden Gruppen, und das Ergebnis war vergleichbar.

## Patentansprüche

1. Mittel zur Verwendung zur Behandlung blutender Wunden von Säugern in Form eines flexiblen Flächengebildes, das aus einem organischen Polymermaterial gebildet wird, welches wenigstens ein partikuläres, kristallines, anorganisches Polyphosphat enthält, wobei das organischen Polymermaterial eine flächenförmige Matrix bildet, in welcher die Partikel des partikulären, kristallinen, anorganischen Polyphosphats in fein verteilter Form vorliegen, wobei das Polyphosphat bei 20 °C eine Löslichkeit in entionisiertem Wasser von weniger als 5 g/L aufweist, wobei das Anion des Polyphosphats im Mittel (Zahlenmittel) mindestens vier Phosphoratome pro Polyphosphat-Anion aufweist.

2. Mittel gemäß Anspruch 1, wobei das Polyphosphat ausgewählt ist unter Alkalimetall-, Erdalkalimetall und Ammoniumpolyphosphaten.

3. Mittel gemäß Anspruch 2 wobei das Polyphosphat ein Natriumpolyphosphat ist.

4. Mittel gemäß einem der vorhergehenden Ansprüche, wobei die Partikel des Polyphosphats einen gewichtsmittleren Teilchendurchmesser, bestimmt durch statische Laserlichtstreuung, im Bereich von 5 bis 40 µm aufweisen.

5. Mittel gemäß einem der vorhergehenden Ansprüche, enthaltend das Polyphosphat in einer Menge im Bereich von 1 bis 30 Gew.-%, bezogen auf das Gesamtgewicht des Mittels.

6. Mittel gemäß einem der vorhergehenden Ansprüche, wobei das organische Polymermaterial thermoplastisch ist.

7. Mittel gemäß Anspruch 6, wobei das organische Polymermaterial als Hauptbestandteil, bezogen auf die darin enthaltenen organischen Polymere, wenigstens ein Polymer enthält, das unter Polyhydroxyalkanoaten, Polylactiden, aliphatisch-aromatischen Polyestern, aliphatisch-aromatischen Polyamiden, Polyolefinen, Polysiloxanen und deren Gemischen ausgewählt ist.

8. Mittel gemäß Anspruch 7, wobei das organische Polymermaterial als Hauptbestandteil, bezogen auf die darin enthaltenen organischen Polymere,
i. ein Gemisch aus wenigstens einem Polylactid und wenigstens einem aliphatisch-aromatischen Polyester; oder
ii. wenigstens einen Copolyester einer Hydroxybuttersäure mit einer Hydroxyalkansäure, die 6 bis 12 C-Atome aufweist, oder ein Gemisch eines solchen Copolyesters mit Polylactid;
enthält.

9. Mittel gemäß einem der Ansprüche 6 bis 8 in Form einer Folie.

10. Mittel gemäß Anspruch 9, wobei das Verhältnis von Foliendicke zu mittlerem Teilchendurchmesser des partikulären Polyphosphats im Bereich von 0,5 : 1 bis 5 : 1 liegt.

11. Mittel gemäß einem der Ansprüche 1 bis 5, wobei das organische Polymermaterial in Form eines Hydrogels auf Basis eines organischen Polymers vorliegt.

12. Mittel gemäß Anspruch 11, wobei das organische Polymermaterial als Hauptbestandteil, bezogen auf die darin enthaltenen organischen Polymere, wenigstens ein Hydrogel-bildendes Polysaccharid, insbesondere wenigstens ein vernetztes carboxyliertes Polysaccharid enthält.

13. Mittel gemäß einem der Ansprüche 11 oder 12, wobei das organische Hydrogelbildende Polysaccharid ein vernetztes Alginat ist.

14. Mittel nach einem der Ansprüche 11 bis 13 in Form eines Pads.

15. Mittel gemäß einem der vorhergehenden Ansprüche, das steril verpackt ist.

16. Mittel gemäß einem der vorhergehenden Ansprüche zur Verwendung bei chirurgischen Eingriffen, insbesondere bei der Operation an inneren Organen.

17. Mittel gemäß einem der vorhergehenden Ansprüche, zur Verwendung bei Patienten, die zuvor mit einem Mittel behandelt wurden, welches die Blutgerinnung herabsetzt oder die an einer Gerinnungsstörung leiden.

18. Verfahren zur Herstellung des Mittels gemäß einem der vorhergehenden Ansprüche, umfassend das Einarbeiten des Polyphosphats und gegebenenfalls weiterer blutstillender Agenzien in das organische Polymermaterial.

## Claims

1. An agent for use in treating bleeding wounds in mammals, in the form of a flexible sheet material formed from an organic polymer material comprising at least one particulate crystalline inorganic polyphosphate, wherein the organic polymer material forms a sheet-like matrix in which the particles of the particulate crystalline inorganic polyphosphate are present in finely divided form, the polyphosphate at 20°C having a solubility in deionized water of less than 5 g/L and the anion of the polyphosphate having an average (number average) of at least four phosphorus atoms per polyphosphate anion.

2. The agent as claimed in claim 1, wherein the polyphosphate is selected from alkali metal polyphosphates, alkaline earth metal polyphosphates, and ammonium polyphosphates.

3. The agent as claimed in claim 2, wherein the polyphosphate is a sodium polyphosphate.

4. The agent as claimed in any of the preceding claims, wherein the particles of the polyphosphate have a mass-median particle diameter, determined by static laser-light scattering, within a range from 5 to 40 µm.

5. The agent as claimed in any of the preceding claims, comprising the polyphosphate in an amount within a range from 1% to 30% by weight based on the total weight of the agent.

6. The agent as claimed in any of the preceding claims, wherein the organic polymer material is thermoplastic.

7. The agent as claimed in claim 6, wherein the organic polymer material comprises as the principal constituent, based on the organic polymers contained therein, at least one polymer selected from polyhydroxyalkanoates, polylactides, aliphatic-aromatic polyesters, aliphatic-aromatic polyamides, polyolefins, polysiloxanes, and mixtures thereof.

8. The agent as claimed in claim 7, wherein the organic polymer material comprises as the principal constituent, based on the organic polymers contained therein,
i. a mixture of at least one polylactide and at least one aliphatic-aromatic polyester; or
ii. at least one copolyester of a hydroxybutyric acid with a hydroxyalkanoic acid having 6 to 12 carbon atoms, or a mixture of such a copolyester with polylactide.

9. The agent as claimed in any of claims 6 to 8 in the form of a film.

10. The agent as claimed in claim 9, wherein the ratio of film thickness to median particle diameter of the particulate polyphosphate is within a range from 0.5:1 to 5:1.

11. The agent as claimed in any of claims 1 to 5, wherein the organic polymer material is in the form of a hydrogel based on an organic polymer.

12. The agent as claimed in claim 11, wherein the organic polymer material comprises as the principal constituent, based on the organic polymers contained therein, at least one hydrogel-forming polysaccharide, in particular at least one crosslinked carboxylated polysaccharide.

13. The agent as claimed in either of claims 11 or 12, wherein the organic hydrogel-forming polysaccharide is a cross-linked alginate.

14. The agent as claimed in any of claims 11 to 13 in the form of a pad.

15. The agent as claimed in any of the preceding claims, which is packaged sterile.

16. The agent as claimed in any of the preceding claims for use in surgical interventions, particularly in operations on internal organs.

17. The agent as claimed in any of the preceding claims, for use in patients who have previously been treated with an agent that reduces blood clotting or who have a coagulopathy.

18. A process for producing the agent as claimed in any of the preceding claims, comprising the incorporation of the polyphosphate and optionally further hemostatic agents into the organic polymer material.

## Revendications

1. Produit destiné à une utilisation pour le traitement de plaies sanglantes de mammifère sous forme d'une structure bidimensionnelle souple, qui est formée d'un matériau polymère organique, lequel contient au moins un polyphosphate inorganique cristallin particulaire, le matériau polymère organique formant une matrice bidimensionnelle, dans laquelle les particules du polyphosphate inorganique cristallin particulaire se présentent sous forme finement divisée, le polyphosphate présentant à 20 °C une solubilité dans l'eau désionisée inférieure à 5 g/l, l'anion du polyphosphate présentant en moyenne (moyenne en nombre) au moins quatre atomes de phosphore par anion polyphosphate.

2. Produit selon la revendication 1, le polyphosphate étant choisi parmi les polyphosphates de métaux alcalins, de métaux alcalino-terreux et d'ammonium.

3. Produit selon la revendication 2, le polyphosphate étant un polyphosphate de sodium.

4. Produit selon l'une des revendications précédentes, les particules du polyphosphate présentant une granulométrie moyenne en poids, déterminée par diffusion de lumière laser statique, dans la plage de 5 à 40 pm.

5. Produit selon l'une des revendications précédentes, contenant le polyphosphate en une quantité dans la plage de 1 à 30 % en poids, par rapport au poids total du produit.

6. Produit selon l'une des revendications précédentes, le matériau polymère organique étant thermoplastique.

7. Produit selon la revendication 6, le matériau polymère organique contenant en tant que constituant principal, pour ce qui est des polymères organiques qui y sont contenus, au moins un polymère qui est choisi parmi les polyhydroxyalcanoates, les polylactides, les polyesters aliphatiques-aromatiques, les polyamides aliphatiques-aromatiques, les polyoléfines, les polysiloxanes et les mélanges de ceux-ci.

8. Produit selon la revendication 7, le matériau polymère organique contenant en tant que constituant principal, pour ce qui est des polymères organiques qui y sont contenus,
i. un mélange d'au moins un polylactide et d'au moins un polyester aliphatique-aromatique ; ou
ii. au moins un copolyester d'un acide hydroxybutyrique et d'un acide hydroxyalcanoïque ayant 6 à 12 atomes de carbone, ou un mélange d'un tel copolyester avec un polylactide.

9. Produit selon l'une des revendications 6 à 8, sous forme d'une feuille.

10. Produit selon la revendication 9, le rapport de l'épaisseur de la feuille à la granulométrie moyenne du polyphosphate particulaire étant dans la plage de 0,5:1 à 5:1.

11. Produit selon l'une des revendications 1 à 5, le matériau polymère organique se présentant sous forme d'un hydrogel à base d'un polymère organique.

12. Produit selon la revendication 11, le matériau polymère organique contenant, en tant que constituant principal, pour ce qui est des polymères organiques qui y sont contenus, au moins un polysaccharide formant un hydrogel, en particulier au moins un polysaccharide carboxylé réticulé.

13. Produit selon l'une des revendications 11 ou 12, le polysaccharide organique formant un hydrogel étant un alginate réticulé.

14. Produit selon l'une des revendications 11 à 13 sous forme d'une compresse.

15. Produit selon l'une des revendications précédentes, qui est emballé dans des conditions stériles.

16. Produit selon l'une des revendications précédentes pour une utilisation lors d'interventions chirurgicales, en particulier lors d'une opération sur des organes internes.

17. Produit selon l'une des revendications précédentes, pour une utilisation chez des patients qui au préalable ont été traités avec un produit qui abaisse la coagulation sanguine, ou qui souffrent d'un trouble de la coagulation.

18. Procédé de fabrication du produit selon l'une des revendications précédentes, comprenant l'incorporation du polyphosphate et éventuellement d'autres agents hémostatiques dans le matériau polymère organique.
